(19) Europäisches Patentamt
European Patent Office
Office européen des brevets

(11) EP 2 431 372 B1

(12) # EUROPEAN PATENT SPECIFICATION

(45) Date of publication and mention of the grant of the patent:
**06.03.2013 Bulletin 2013/10**

(51) Int Cl.:
***C07F 7/08*** *(2006.01)* ***C08G 65/336*** *(2006.01)*
***C08G 77/48*** *(2006.01)*

(21) Application number: **11005484.8**

(22) Date of filing: **30.03.2007**

(54) **Agrochemical composition comprising a hydrolysis resistant organomodified silylated surfactant**

Agrochemische Zusammensetzung enthaltend ein hydrolysebeständiges organomodifiziertes silyliertes Tensid

Composition agrochimique comprenant un tensioactif silylé organomodifié résistant à l’hydrolyse

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **21.04.2006 US 379592**

(43) Date of publication of application:
**21.03.2012 Bulletin 2012/12**

(62) Document number(s) of the earlier application(s) in accordance with Art. 76 EPC:
**07754424.5 / 2 016 083**

(73) Proprietor: **Momentive Performance Materials Inc.**
**New York 12211 (US)**

(72) Inventors:
- **Leatherman, Mark, D.**
  **Stamford, CT06907 (US)**
- **Peng, Wenqing**
  **Shangai 201203 (CN)**
- **Policello, George, A.**
  **Ossining, NY 10562 (US)**
- **Rajarman, Suresh, K.**
  **Newburg, NY 12550 (US)**
- **Wagner, Roland**
  **53227 Bonn (DE)**
- **Xia, Zijun**
  **Pudong, Shanghai 20123 (CN)**

(74) Representative: **Laufhütte, Dieter et al**
**Lorenz-Seidler-Gossel**
**Widenmayerstrasse 23**
**80538 München (DE)**

(56) References cited:
**EP-A- 0 367 381** **WO-A-2007/044552**
**WO-A1-94/22311** **WO-A1-2005/013693**
**DE-A1- 4 433 139** **DE-C1- 4 320 920**
**US-B1- 6 211 284**

- **MAKI H ET AL: "SYNTHESES AND PROPERTIES OF SURFACTANTS CONTAINING ORGANOMETALS: IVORGANO SILICON", JOURNAL OF THE AMERICAN OIL CHEMISTS’ SOCIETY, AOCS PRESS, CHAMPAIGN, IL, US, vol. 46, no. 12, 1969, pages 635-638, XP000882184, ISSN: 0003-021X**
- **R. WAGNER ET AL.: "Silicon-Modified Carbohydrate Surfactants V: Wetting Behaviour of Low-Molecular-Weight Siloxane, Carbosilane, Silane and Polysilane Precursors on Low-Energy Surfaces", APPLIED ORGANOMETALLIC CHEMISTRY, vol. 11, 1997, pages 645-657, XP002445519,**
- **HILL R M: "Dynamics of surfactant enhanced spreading", COMPENDEX,, 1 January 1998 (1998-01-01), XP002560623,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 431 372 B1

## Description

The present invention relates to agrochemical compositions comprising organomodified silylated surfactant compositions that exhibit resistance to hydrolysis over a wide pH range. More particularly such hydrolysis-resistant organomodified silylated surfactants have a resistance to hydrolysis between a pH of about 2 to a pH of about 12

BACKGROUND OF THE INVENTION

The topical application of liquid compositions to the surfaces of both animate and inaminate objects to effect a desired change involve the processes of controlling wetting, spreading, foaming, detergency, and the like. When used in aqueous solutions to improve the delivery of active ingredients to the surface being treated, trisiloxane-type compounds have been found to be useful in enabling the control of these processes to achieve the desired effect However, the trisiloxane compounds may only be used in a narrow pH range, ranging from a slightly acidic pH of 6 to a very mildly basic pH of 7.5. Outside this narrow pH range, the trisiloxane compounds are not stable to hydrolysis, undergoing rapid decomposition.

SUMMARY OF THE INVENTION

The present invention provides for agrochemical compositions comprising an organomodified silylated surfactant compound or compositions thereof useful as a surfactant having the general formula:

$(R^1)(R^2)(R^3)Si\ -R^4-Si(R^5)(R^6)(R^7)$

wherein

$R^1$, $R^2$, $R^3$, $R^5$, and $R^6$ are each independently selected from the group consisting of 1 to 6 carbon atom monovalent hydrocarbon radicals, aryl, and a hydrocarbon group of 7 to 10 carbons containing an aryl group;

$R^4$ is a hydrocarbon group of 1 to 3 carbons.

$R^7$ is an alkyleneoxide group of the general formula:

$R^8(C_2H_4O)_d(C_3(C_3H_6O)_e(C_4H_8O)_fR^9$

where $R^8$ is a divalent linear or branched hydrocarbon radical having the structure:

$-CH_2-CH(R^{10})(R^{11})_gO-$

where $R^{10}$ is H or methyl; $R^{11}$ is a divalent alkyl radical of 1 to 6 carbons where the subscript g may be 0 or 1;

$R^9$ is selected from the group consisting of H, monovalent hydrocarbon radicals of 1 to 6 carbon atoms and acetyl, subject to the limitation that the subscripts d, e and f are zero or positive and satisfy the following relationships:

$$2 \leq d + e + f \leq 20 \text{ with } d \geq 2.$$

DETAINED DESCRIPII0N OF THE INVENTION

As used herein, integer values of stoichiometric subscripts refer to molecular species and non-integer values of stoichiometric subscripts refer to a mixture of molecular species on a molecular weight average basis, a number average basis or a mole fraction basis.

The present invention provides for agrochemical compositions comprising an organomodified silylated surfactant compound or compositions thereof useful as a surfactant having the general formula:

$(R^1)(R^2)(R^3)Si-R^4-Si(R^5)(R^6)(R^7)$

whereon

$R^1$, $R^2$, $R^3$, $R^5$, and $R^6$ are each independently selected from the group consisting of 1 to 6 carbon atom monovalent hydrocarbon radicals; and a monovalent hydrocarbon group of 7 to 10 carbon atoms containing an aryl group;

$R^4$ is a hydrocarbon group of 1 to 3 carbons;

$R^7$ is an alkyleneoxide group of the general formula:

$$R^8(C_2H_4O)_d(C_3H_6O)_e(C_4H_8O)_fR^9$$

where $R^6$ is a divalent linear or branched hydrocarbon radical having the structure:

$$-CH_2-CH(R^{10})(R^{11})_gO-$$

where $R^{10}$ is H or methyl; $R^{11}$ is a divalent alkyl radical of 1 to 6 carbons where the subscript g may be 0 or 1;

$R^9$ is selected from the group consisting of H, monovalent hydrocarbon radicals of 1 to 6 carbon atoms and acetyl subject to the limitation that the subscripts d, e and f are zero or positive and satisfy the following relationships:

$$2 \leq d + e + f \leq 20 \text{ with } d \geq 2.$$

Preferred embodiments

One preferred embodiment of the organomodified Silylated surfactant is where $R^1$, $R^2$, $R^3$, $R^5$, and $R^6$ are each independently selected from the group consisting of 1 to 6 monovalent hydrocarbon radicals, and a hydrocarbon group of 7 to 10 carbons containing or substituted with an aryl group; preferably a hydrocarbon group of 7 to 8 carbons containing an aryl group and more preferably a hydrocarbon group of 7 carbons containing an aryl group;
$R^4$ is a hydrocarbon group of 1 to 3 carbons;
$R^7$ is an alkyleneoxide group of the general formula:

$$R^8(C_2H_4O)_4(C_3H_6O)_e(C_4H_8O)_fR^9$$

where $R^8$ is a divalent linear or branched hydrocarbon radical having the structure.

$$-CH_2-CH(R^{10})(R^{11})_gO-$$

$R^{10}$ is H or methyl; $R^{11}$ is a divalent alkyl radical of 1 to 6 carbon atoms, more preferably 1 to 2 carbon atoms, where the subscript g may be 0 or 1;
$R^9$ is selected from the group consisting of H, monovalent hydrocarbon radicals of 1 to 6 carbon atoms and acetyl; more preferably H or methyl, where $R^7$ is subject to the limitation that the subscripts d, e and f are zero or positive and satisfy the following relationships:

$2 \leq d+e+f \leq 20$ with $d \geq 2$; with d preferably ranging from 3 to 20, and more preferably from 5 to 8; with e preferably anging from 0 to 10; and more preferably 0 to 5; with f preferably ranging from 0 to 8, and more preferably from 0 to 4.

Another preferred embodiment of the organomodified Silylated surfactant is where $R^1$, $R^2$, $R^9$, $R^5$ and $R^6$ are each methyl; $R^4$ is a hydrocarbon group of 1 to 3 carbon atoms; $R^{10}$ is H; $R^{11}$ is methyl, where the subscript g is 1. $R^9$ is H or methyl. Subscripts d, e and f are zero or positive and satisfy the following relationships:
$2 \leq d+e+f \leq 17$ with $d \geq 2$; preferably d ranges from 3 to 9, and more preferably from 5 to 8; preferably e ranges from 0 to 5; and more preferably 0 to 3; preferably f is 0 to 2.
Yet another preferred embodiment of the organomodified silylated surfactant is where $R^1$, $R^2$, $R^3$ $R^5$, and $R^6$ are each methyl; $R^4$ is a hydrocarbon group of 1 or 2 carbon atoms; $R^{10}$ is H; $R^{11}$ is methyl, where the subscript g is 1; the subscript d ranges from 6 to 8, both e and f are 0.
One method of producing the composition of the present invention is to react a molecule of the following formula:

$$(R^1)(R^2)(R^3)Si\ -R^4-Si(R^5)(R^6)(R^{12})$$

where $R^{12}$ is H, wherein the definitions and relationships are later defined and consistent with those defined above, under hydrosilylation conditions, with an olefinically modified polyalkyleneoxide, such as allyloxypolyethyleneglycol, or methallyloxypolyalkyleneoxide, which are incorporated here as examples, and not set forth to limit other possible olefinically modified alkyleneoxide components. As used herein the phrase "olefinically modified polyalkyleneoxide" is defined as a molecule possessing one or more alkyleneoxide groups containing one or more, terminal or pendant, carbon-carbon double bonds. The polyether is an olefinically modified polyalkyleneoxide (hereinafter referred to as "polyether") is described by the general formula:

$$CH_2{=}CH(R^{10})(R^{11})_gO(C_2H_4O)_d(C_3H_6O)_e(C_4H_gO)_fR^9$$

where

$R^{10}$ is H or methyl; $R^{11}$ is a divalent alkyl radical of 1 to 6 carbons where the subscript g may be 0 or 1; $R^9$ is H, a monofunctional hydrocarbon radical of 1 to 6 carbons, or acetyl. When the polyether is composed of mixed oxyalkyleneoxide groups (i.e. oxyethylene, oxypropylene and oxybutylene) the units may be blocked, or randomly distributed. Illustrative examples of blocked configurations are: -(oxyethylene)$_a$(oxypropylene)$_b$-; -(oxybutylene)$_c$(oxyethylene)$_a$-; -(oxypropylene)$_b$(oxyethylene)$_a$(oxybutylene)$_c$-, and the like.

Non-limiting illustrative examples of the olefinically modified polyalkyleneoxide are:

$CH_2{=}CHCH_2O(CH_2CH_2O)_8H$; $CH_2{=}CHCH_2O(CH_2CH_2O)_8CH_3$;

$CH_2{=}CHCH_2O(CH_2CH_2O)_4(CH_2CH(CH_3)O)_5H$;

$CH_2{=}CHO(CH_2CH_2O)_5(CH_2CH(CH_3)O)_5H$;

$CH_2{=}C(CH_3)CH_2O(CH_2CH_2O)_4(CH_2CH(CH_3)O)_5C(O)_5C({=}O)CH_3$;

$CH_2{=}CHCH_2O(CH_2CH_2O)_5(CH_2CH(CH_3)O)_2(CH_2CH(CH_2CH_3)O)_2H$

Polyether-modified carbosilanes are straightforwardly prepared through the use of a hydrosilylation reaction to graft the olefinically modified (i.e. vinyl, allyl or methallyl) polyalkyleneoxide onto the hydride (SiH) intermediate of the trisiloxane of the present invention.

Precious metal catalysts suitable for making polyether-substituted silanes are also well known in the art and comprise complexes of rhodium, ruthenium, palladium, osmium, iridium, and /or platinum. Many types of platinum catalysts for this SiH-olefin addition reaction are known and such platinum catalysts may be used to generate the compositions of the present invention. The platinum compound can be selected from those having the formula ($PtCl_2$Olefin) and 14($PtCl_3$Olefin) as described in U.S. Pat No. 3,159,601, hereby incorporated by reference. A further platinum containing material can be a complex of chloroplatinic acid with up to 2 moles per gram of platinum of a member selected from the class consisting of alcohols, ethers, aldehydes and mixtures thereof as described in U.S. Pat No. 3,220,972, hereby incorporated by reference. Yet another group of platinum containing materials useful in this present invention is described in U.S. Pat Nos. 3,715,334; 3,775,452 and 3,814,730 (Karstedt). Additional background concerning the art may be found in J.L. Spier, "Homogeneous Catalysis of Hydrosilation by Transition Metals", in Advances in Organometallic Chemistry, volume 17, pages 407 through 447, F.G.A. Stone and R. West editors, published by Academic Press (New York, 1979). Those skilled in the art can easily determine an effective amount of platinum catalyst Generally an effective amount ranges from about 0.1 to 50 parts per million of the total organomodified silylated surfactant composition.

Pesticide-Agriculture, Horticulture Turf, Ornamental and Forestry

Many pesticide applications require the addition of an adjuvant to the spray mixture to provide wetting and spreading on foliar surfaces. Often that adjuvant is a surfactant which can perform a variety of functions; such as increasing spray droplet retention on difficult to wet leaf surfaces, enhance spreading to improve spray coverage, or to provide penetration of the herbicide into the plant cuticle. These adjuvants are provided either as a tank-side additive or used as a component in pesticide formulations.

Typical uses for pesticides include agricultural, horticultural, turf, ornamental, home and garden, veterinary and forestry applications.

The pesticidal compositions of the present invention also include at least one pesticide, where the organomodified silylated surfactant of the present invention is present at an amount sufficient to deliver between 0.005% and 2% to the final use concentration, either as a concentrate or diluted in a tank mix. Optionally the pesticidal composition may include excipients, cosurfactants, solvents, foam control agents, deposition aids, drift retardants, biologicals, micronutrients, fertilizers and the like. The term pesticide means any compound used to destroy pests, e.g., rodenticides, insecticides, miticides, fungicide, and herbicides. Illustrative examples of pesticides that can be employed include, but are not limited to, growth regulators, photosynthesis inhibitors, pigment inhibitors, mitotic disrupters, lipid biosynthesis inhibitors, cell wall inhibitors, and cell membrane disrupters. The amount of pesticide employed in compositions of the invention varies with the type of pesticide employed. More specific examples of pesticide compounds that can be used with the compositions of the invention are, but not limited to, herbicides and growth regulators, such as phenoxy acetic acids, phenoxy propionic acids, phenoxy butyric acids, benzoic adds, triazines and s-triazines, substituted ureas, uracils, bentazon, desmedipham, methazole, phenmedipham pyridate, amitrole, clomazone, fluridone, norflurazone, dinitroanilines, isopropalin, oryzalin, pendimethalin, prodiamine, trifluralin, glyphosate, sulfonylureas, imidezolinones, clethodim, diclofop-methyl, fenoxaprop-ethyl, fluazifop-p-butyl, haloxyfop-methyl, quizalofop, sethoxydim, dichlobenil, isoxaben, and bipyridylium compounds.

Fungicide compositions that can be used with the present invention include, but are not limited to, aldimorph, tridemorph dodemorph, dimethomorph; flusilazol, azaconazole, cyproconazole, epoxiconazole, furconaxole, propiconazole; tebuconazole and the like; imazalil, thiophanate, benomyl carbendazim, chlorothialonil, dicloran, trifloxystrobin, fluoxystrobin, dimoxystrobin, azoxystrobin, furcaranil, prochloraz, flusulfamide, famoxadone, captan, maneb, mancozeb, dodicin, dodine, and metalaxyl.

Insecticide, larvacide, mititicide and ovacide compounds that can be used with the composition of the present invention, but not limited to, Bacillus thuringiensis, spinosad, abamectin, doramectin, lepimectin, pyrethrins, carbaryl, primicarb, aldicarb, methomyl, amitraz, boric acid, chlordimeform, novaluron, bistrifluron, triflumuron, diflubenzuron, imidacloprid, diazinon, acephate, endosulfan, kelevan, dimethoate, azinphos-ethyl, azinphos-methyl, izoxathion, chlorpyrifos, clofenbezine, lambda-cyhalothrin, permethrin, bifenthrin, cypermethrin and the like.

The pesticide may be a liquid or a solid. If a solid, it is preferable that it is soluble in a solvent, or the organomodified silylated surfactant of the present invention, prior to application, and the organomodified silylated surfactant may act as a solvent, or surfactant for such solubility or additional surfactants may perform this function.

<u>Agricultural Excipients:</u>

Buffers, preservatives and other standard excipients known in the art also may be included in the composition

Solvents may also be included in compositions of the present invention. These solvents are in a liquid state at room temperature. Examples include water, alcohols, aromatic solvents, oils (i.e. mineral oil, vegetable oil, silicone oil, and so forth), lower alkyl esters of vegetable oils, fatty acids, ketones, glycols, polyethylene glycols, diols, paraffinics, and so forth. Particular solvents would be 2,2,4-trimethyl, 1-3-pentane diol and alkoxylated (especially ethoxylated) versions thereof as illustrated in US Patent No. 5,674,832 or N-methyl-pyrrilidone.

<u>Cosurfactants:</u>

Moreover, other cosurfactants, which have short chain hydrophobes that do not interfere with superspreading as described in US Patent No. 5,558,806.

The cosurfactants useful herein include nonionic, cationic, anionic, amphoteric, zwitterionic, polymeric surfactants, or any mixture thereof. Surfactants are typically hydrocarbon based, silicone based or fluorocarbon based.

Useful surfactants include alkoxylates, especially ethoxylates, containing block copolymers including copolymers of ethylene oxide, propylene oxide, butylene oxide, and mixtures thereof; alkylarylalkoxylates, especially ethoxylates or propoxylates and their derivatives including alkyl phenol ethoxylate; arylarylalkoxylates, especially ethoxylates or propoxylates, and their derivatives; amine alkoxylates, especially amine ethoxylate; fatty acid alkoxylates; fatty alcohol alkoxylates; alkyl sulfonates; alkyl benzene and alkyl naphthalene sulfonates; sulfated fatty alcohols, amines or acid amides; acid esters of sodium isethionate; esters of sodium sulfosuccinate; sulfated or sulfonated fatty acid esters; petroleum sulfonates; N-acyl sarcosinates; alkyl polyglycosides; alkyl ethoxylated amines; and so forth.

Specific examples include alkyl acetylenic diols (SURFONYL- Air Products), pyrrilodone based surfactants (e.g., SURFADONE - LP 100 - ISP), 2-ethyl hexyl sulfate, isodecyl alcohol ethoxylates (e.g., RHODASURF DA 530 - Rhodia), ethylene diamine alkoxylates (TETRONICS - BASF), and ethylene oxide/propylene oxide copolymers (PLURONICS -BASF) and Gemini type surfactants (Rhodia).

Preferred surfactants include ethylene oxide/propylene oxide copolymers (EO/PO); amine ethoxylates; alkyl polyglycosides; oxo-tridecyl alcohol ethoxylates, and so forth.

In a preferred embodiment, the agrochemical composition of the present invention further comprises one or more agrochemical ingredients. Suitable agrochemical ingredients include, but not limited to, herbicides, insecticides, growth regulators, fungicides, miticides, acaricides, fertilizers, biologicals, plant nutritionals, micronutrients, biocides, paraffinic mineral oil, methylated seed oils (i.e. methylsoyate or methylcanolate), vegetable oils (such as soybean oil and canola oil), water conditioning agents such as Choice® (Loveland Industries, Greeley, CO) and Quest (Helena

Chemical, Collierville, TN), modified clays such as Surround® (Englehard Corp.,), foam control agents, surfactants, wetting agents, dispersants, emulsifiers, deposition aids, antidrift components, and water.

Suitable agrochemical compositions are made by combining, in a manner known in the art, such as by mixing, one or more of the above components with the organomodified silylated surfactant of the present invention, either as a tank-mix, or as an "in-can" formulation The term "tank-mix" means the addition of at least one agrochemical to a spray medium, such as water or oil, at the point of use. The term "in-can" refers to a formulation or concentrate containing at least one agrochemical component. The "in-can" formulation may then diluted to use concentration at the point of use, typically in a tank-mix, or it may be used undiluted.

EXPERIMENTAL

The hydride intermediates for the organomodified silylated surfactant compositions of the present invention, as well as comparative compositions were prepared as described in the following examples.

Preparation Example 1

(Trimethylsilylmethyl)dimethylsilane (Figure 1, Structure 1).

The Grignard reagent of trimethylchloromethylsilane (TMCMS) was prepared by reaction of 123 g (0.1 mol) TMCMS and 288 g (0.12 mol) magnesium chips in THF (50 mL). The Grignard reagent was then added dropwise into 9.46 g (0.1 mol)dimethylchlorosilane (DMCS), which dissolved in THF (50 mL). The mixture was stirred at room temperature overnight and quenched with 20 mL HCl-acidified water, and then extracted with diethylether (100 mL). The organic layer was washed with distilled water three times and dried with anhydrous sodium sulfate. The mixture was purified by distillation at 118-119 °C to yield 13.0 g (89%) (trimethylsilylmethyl)dimethylsilane product as a dear, colorless fluid.

**Figure 1 - Reaction Sequence for Preparation of Hydride Intermediate**

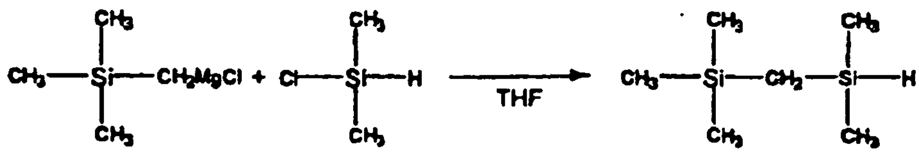


**Structure 1**

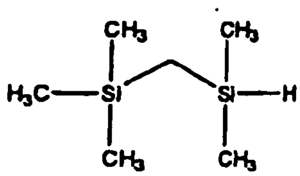

Preparation Example 2

((2-Trimethylsilyl)ethyl)dimethylsilane (Figure 2, Structure 2).

10g (0.1 mol) trimethylvinylsilane (TMVS), 9.46g (0.1 mol) dimethylchlorosilane (DMCS) and 10 $\mu$l platinum (0)-1,3-divinyl-1,1,3,3-tetramethyldisiloxane complex (0.1 M in xylene) were placed into a 100 mL three-necked round bottom flask equipped with $N_2$ inlet and reflux condenser. The mixture was stirred at room temperature for 30 min and heated to 70 °C for 2h. The reaction was monitored by $^1H$ NMR. After cooling down to room temperature, 50 mL of THF was introduced and the solution was cooled to -80 °C. 1.00g $LiAlH_4$ was added to the solution and stirred until the mixture warmed up to room temperature. The mixture was further stirred at room temperature overnight. 10 mL of acidified water was added to quench the reaction, and the organic layer was separated, washed with water three times and dried over anhydrous sodium sulfate. The mixture was purified by distillation, and 12.7 g (yield 79.2%) product was collected at b.p.140-141 °C as a clear colorless fluid.

## Figure 2 - Reaction Sequence for Preparation of Hydride Intermediate

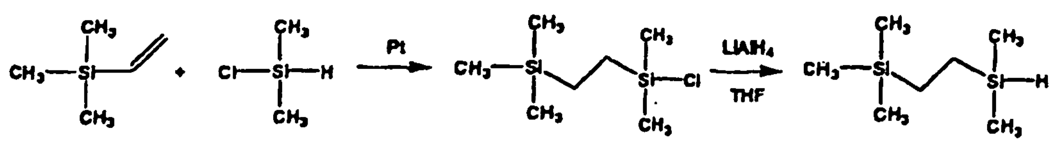


## Structure 2

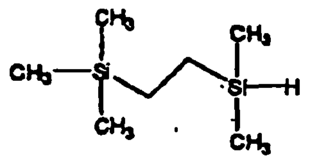

Preparation Example 3

((3-Trimethylsilyl)propyl)dimethylsilane (Figure 3, Structure 3).

11.4g (0.1 mol) trimethylallylsilane, 9.5g (0.1 mol) dimethylchlorosilane (DMCS) and 10 μl platinum(0)-1,3-divinyl-1,1,3,3-tetramethyldisiloxane complex (0.1 M in xylene) were placed into a 100 mL three-necked round bottom flask equipped with $N_2$ inlet and condenser. The mixture was stirred at room temperature for 30 min and heated to 70 °C for 2h. The reaction was monitored by $^1H$ NMR. After cooling down to room temperature, 50 mL of THF was introduced and the solution was cooled to -80 °C. 1.00g $LiAlH_4$ was added to the solution and stirred until the mixture warmed up to room temperature. The mixture was further stirred at room temperature overnight.10 mL of acidified water was added to quench the reaction, and the organic layer was separated, washed with water three times and dried over anhydrous sodium sulfate. The mixture was purified by vacuum distillation, and 123 g (yield 70.7%) product was collected at b.p. 60-61°C 133-267 Pa (1-2 mmHg) as a clear colorless fluid.

## Figure 3 - Reaction Sequence for Preparation of Hydride Intermediate

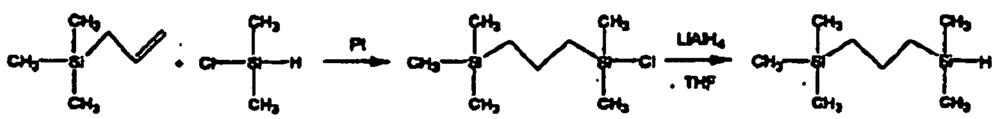


## Structure 3

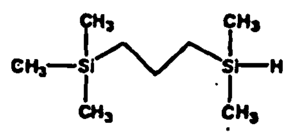

Preparation Example 4

The hydride intermediates of Examples 1-3 were further modified with various allylpolyalkyleneoxides to yield the organomodified silylated surfactant compositions of the present invention (Table 1), as well as comparative trisiloxane surfactants (From Table 2).

The organomodified silylated surfactant compositions of the present invention were prepared by conventional methods of platinum-mediated hydrosilylation, as described in Bailey, U.S. Patent 3,299,112, herein incorporated by

reference.

Table 1 provides a description of the compositions of the present invention. These compositions are described by the general structure:

$$(CH_3)_3Si(CH_2)_mSi(CH_3)_2(CH_2CH_2CH_2O(CH_2CH_2O)_nR^9),$$

wherein m, n and $R^9$ are described in Table 1.

Table 1-Description of Examples of Organomodified Silylated Surfactant Compositions of the Present Invention

| I.D. | m | n | R |
|---|---|---|---|
| 1 | 1 | 7.5 | $CH_3$ |
| 2 | 2 | 7.5 | $CH_3$ |
| 3 | 3 | 7.5 | $CH_3$ |
| 4 | 1 | 7.5 | H |
| 5 | 2 | 7.5 | H |
| 6 | 1 | 4 | H |
| 7 | 2 | 4 | H |
| .8 | 1 | 11 | H |
| 9 | 2 | 11 | H |

Table 2 provides a description of the comparative trisiloxane and organosilicone polyether based surfactants of the general structure:

$$MD_x D''_Y M$$

where M = $(CH_3)_3SiO_{0.5}$; D = O $Si(CH_3)_2$; and

$$D'' = OSi(CH_3)CH_2CH_2CH_2O\text{-}(CH_2CH_2O)_aR^{13}$$

Table 2- Composition of Comparative Organosilicone Polyether Surfactants

| | | | Polyether Group | |
|---|---|---|---|---|
| ID. | X | Y | a | $R^{13}$ |
| A | 0 | 1 | 7.5 | $CH_3$ |
| B | 0 | 1 | 7.5 | H |
| C | 20 | 3 | 7.5 | $CH_3$ |

Additionally, comparative sample OPE (Octylphenolethoxylate, containing 10 polyoxyethylene units) is a non-silicone organic surfactant This product is available as Triton® X-100 from Dow Chemical Company, Midland, MI.

Example 5

This example demonstrates the ability of the organomodified silylated surfactant compositions of the present invention to reduce aqueous surface tension, thereby showing utility as surfactants. Surface tension was measured using a Kruss surface tensiometer, with a sand blasted platinum blade as the sensor. Solutions of the various components were prepared at 0.1 wt% in 0.005M NaCl water (deionized), as an equilibrium aid.

Table 3 shows that solutions of these unique compositions provide a significant reduction in surface tension relative to the conventional surfactant

The compositions of the present invention also provide spreading properties similar to the comparative trisiloxane surfactants (A, B). Additionally, organomodified silylated surfactants of the present invention provide improved spreading relative to the conventional silicone polyether (C) and conventional organic surfactant product OPE.

Spreading was determined by applying a 10 μL droplet of surfactant solution to polystyrene Petri dishes (Fisher Scientific) and measuring the spread diameter (mm) after 30 seconds, at a relative humidity between 50 and 70% (at 22 to 25°C). The solution was applied with an automatic pipette to provide droplets of reproducible volume. Deionized water that was further purified with a Millipore filtration system was used to prepare the surfactant solutions.

Table 3-Surface Tension and Spreading Properties

| | Surface Tension | Spread Diameter (mm) Weight % Surfactant | | | |
|---|---|---|---|---|---|
| I.D. | mN/m | 0.05% | 0.1% | 0.3% | 0.5% |
| 1 | 24.2 | 24 | 41 | 43 | 45 |
| 2 | 24.6 | 27 | 44 | 44 | 45 |
| 3 | 23.8 | 28 | 44 | 44 | 39 |
| 4 | 24.4 | 23 | 36 | 36 | 22 |
| 5 | 23.6 | 27 | 44 | 40 | 33 |
| 6 | 22.5 | 40 | 45 | 53 | 47 |
| 7 | 22.7 | 30 | 42 | 48 | 49 |
| 8 | 27.7 | nd | 7 | nd | 7 |
| 9 | 26.7 | nd | 7 | nd | 7 |
| A | 20.9 | 34 | 53 | 51 | 25 |
| B | 20.6 | 37 | 53 | 50 | 35 |
| C | 23.6 | nd | nd | nd | 6 |
| OPE | 31.8 | nd | 9 | nd | 10 |

Example 6

Hydrolytic stability was determined for representative compositions used in the present invention using HPLC Solutions of the various compositions were prepared at 0.5 wt% over a pH range from pH 2 to pH 12, and monitored by HPLC for decomposition as a function of time.

Analytical Method:

The samples were analyzed by a reverse-phase chromatographic technique using the experimental conditions listed in Table 4.

Table 4-Solvent Gradient for HPLC Method

| Time (min.) | % Methanol | %Water | % Isopropanol |
|---|---|---|---|
| 0.0 | 70 | 30 | 0 |
| 15.0 | 100 | 0 | 0 |
| 20.0 | 50 | 0 | 50 |
| 20.1 | 70 | 30 | 0 |
| 25.0 | 70 | 30 | 0 |

| | |
|---|---|
| Detector: | ELSD/LTA (Evaporative Light Scattering with Low Temperature Adapter |
| Conditions: | 30°C, 1.95 SLPM $N_2$ |
| Column: | Phenomenex LUNA C18 end cap, 5 micron, 75x4.6 mm |
| Flow Rate: | 1.0 mL/min. |
| Inj. Volume: | 10 microlitres |

(continued)

Sample: 0.050 g/mL in methanol

Tables 5-7 demonstrate that the compositions of the present invention provide improved resistance to hydrolytic decomposition relative to the standard comparative siloxane-based surfactant Siloxane A, under similar pH conditions.

Comparative siloxane A shows rapid hydrolysis at ≤ pH 5 and > pH 9, while the organomodified silylated surfactants of the present invention demonstrate a higher resistance to hydrolysis under the same conditions.

Table 5-Hydrolytic Stability of Organomodified Silylated Surfactants by HPLC

| | | Stability: % Silylated Surfactant Remaining | | | | | | |
|---|---|---|---|---|---|---|---|---|
| LD. | Time | pH2 | pH4 | pH 5 | pH7 | pH9 | pH 10 | pH 12 |
| 1 | 24h | nd | 100 | 100 | 100 | 100 | 100 | nd |
| | 1 wk | 100 | 100 | 100 | 100 | 100 | 100 | 77 |
| | 2 wk | nd | 100 | 100 | 100 | 100 | 100 | nd |
| | 3 wk | 100 | nd | Nd | nd | nd | nd | 73 |
| | 4 wk | nd | 100 | 100 | 100 | 100 | 100 | nd |
| | 7 wk | 89 | 100 | 100 | 100 | 100 | 100 | 76 |
| | 12 wk | 95 | 100 | 100 | 100 | 100 | 100 | 76 |
| | 19 wk | 95 | 100 | 100 | 100 | 100 | 100 | 72 |
| | 30 wk | 73 | 100 | 100 | 100 | 100 | 100 | 74 |

Table 6-Hydrolytic Stability of Organomodified Silylated Surfactants by HPLC

| | | Stability: % Silylated Surfactant Remaining | | | | | | |
|---|---|---|---|---|---|---|---|---|
| I.D. | Time | pH2 | pH4 | pH5 | pH7 | pH9 | pH10 | pH12 |
| 2 | 24h | nd | 100 | 100 | 100 | 100 | 100 | nd |
| | 1 wk | 100 | 100 | 100 | 100 | 100 | 100 | 77 |
| | 2 wk | nd | 100 | 100 | 100 | 100 | 100 | nd |
| | 3 wk | 100 | nd | nd | nd | nd | nd | 77 |
| | 4 wk | nd | 100 | 100 | 100 | 100 | 100 | nd |
| | 7 wk | 100 | 100 | 100 | 100 | 100 | 100 | 74 |
| | 12 wk | 86 | 100 | 100 | 100 | 100 | 100 | 74 |
| | 19 wk | 79 | 87 | 100 | 100 | 100 | 100 | 77 |
| | 30 wk | 73 | 79 | 90 | 100 | 94 | 97 | 75 |

Table 7-Hydrolytic Stability of Comparative Siloxane-Based Surfactants by HPLC

| | | Stability: % Siloxane Surfactant Remaining | | | | |
|---|---|---|---|---|---|---|
| I.D. | Time | pH4 | pH 5 | pH7 | pH9 | pH10 |
| A | 24h | 50 | 93 | 100 | 95 | 75 |
| | 48h | 22 | 85 | 100 | 88 | 52 |
| | 1 wk | 0 | 58 | 100 | 72 | 12 |

Example 7

Unlike traditional siloxane based surfactants, which are subject to rapid hydrolysis under acidic and basic conditions (≤pH 5 and ≥ pH 9), the organomodified silylated surfactants of the present invention provide increased resistance to hydrolysis relative to traditional trisiloxane alkoxylates (Comparative Example A). An artifact of hydrolysis is observed as a reduction in spreading properties over time. Therefore, solutions of the organomodified silylated surfactants of the present invention, as well as comparative surfactants, were prepared at desired use levels and pH. Spreading was determined as a function of time to illustrate resistance to hydrolysis.

Table 8 is an illustrative example of a traditional organomodified trisiloxane ethoxylate surfactant, which exhibits decreased spreading performance with time as a function of hydrolytic decomposition over a pH range from pH 3 to pH 10. Here a 0.4 wt% solution of product A was prepared at pH 3, 4, 5 and 10. Spreading was determined by applying a 10 μL droplet of surfactant solution to polyacetate film (USI, "Crystal Clear Write on Film") and measuring the spread diameter (mm) after 30 seconds, at a relative humidity between 50 and 70% (at 22 to 25°C). The solution was applied with an automatic pipette to provide droplets of reproducible volume. Deionized water that was further purified with a Millipore filtration system was used to prepare the surfactant solutions.

Table 8-Effect of pH on Spreading Properties Vs. Time

| | Spread Diameter (mm) | | | | |
|---|---|---|---|---|---|
| Time | Product | pH3 | pH4 | pH5 | pH10 |
| 0h | A | 34 | 28 | 29 | 27 |
| 1h | A | 39 | 37 | 27 | 33 |
| 2h | A | 36 | 30 | 33 | 33 |
| 4h | A | 41 | 28 | 28 | 29 |
| 6h | A | 16 | 27 | 27 | 28 |
| 8h | A | 12 | 31 | 29 | 27 |
| 24h | A | 12 | 32 | 25 | 25 |
| 48h | A | 10 | 41 | 25 | 33 |
| 5 days | A | 7 | 30 | 26 | 36 |
| 7 days | A | 6 | 17 | 28 | 25 |
| 14 days | A | 7 | 7 | 37 | 15 |

Table 9 is an illustrative example of an organomodified silylated surfactant of the present invention, where product No. 2, a superspreader, has improved resistance to hydrolysis, over a pH range from pH 3 to pH 10 relative to a traditional trisiloxane ethoxylate surfactant (Product A). As mentioned above, resistance to hydrolysis was observed by monitoring the spreading properties over time. Here a 0.1 wt% solution was prepared at pH 3, 4, 5 and 10. Spreading was determined by applying a 10 μL droplet, of surfactant solution to polystyrene Petri dishes (Fisher Scientific) and measuring the spread diameter (mm) after 30 seconds, at a relative humidity between 50 and 70 % (at 22 to 25°C). The solution was applied with an automatic pipette to provide droplets of reproducible volume. Deionized water that was further purified with a Millipore filtration system was used to prepare the surfactant solutions.

Table 9-Effect of pH on Spreading Properties Vs. Time

| | Spread Diameter (mm) | | | | |
|---|---|---|---|---|---|
| Time | Product | pH 3 | pH4 | pH5 | pH 10 |
| 0h | 2 | 39 | 39 | 41 | 27 |
| 24h | 2 | 37 | 38 | 37 | 35 |
| 48h | 2 | 39 | 39 | 36 | 38 |
| 72h | 2 | 39 | 38 | 39 | 35 |
| 1 week | 2 | 38 | 39 | 40 | 36 |

(continued)

| | Spread Diameter (mm) | | | | |
|---|---|---|---|---|---|
| Time | Product | pH 3 | pH4 | pH5 | pH 10 |
| 2 weeks | 2 | 39 | 37 | 39 | 39 |
| 1 month | 2 | 40 | 39 | 40 | 39 |
| 2 months | 2 | 43 | 41 | 41 | 41 |
| 3 months | 2 | 39 | 40 | 37 | 45 |
| 6 months | 2 | 43 | 40 | 44 | 41 |
| 12 months | 2 | 45 | 38 | 42 | 41 |

Example 8

The impact of other ingredients on spreading was determined by blending the organomodified silylated surfactant of the present invention, with a conventional organic based co-surfactant The co-surfactants are described in Table 10.

Blends were prepared as physical mixtures where the weight fraction of the silylated surfactant is represented by $\alpha$ (alpha), indicating that the co-surfactant makes up the balance of the blend ratio. For example when $\alpha$ = 0 this indicates that the composition contains 0% of the silylated surfactant component and 100% co-surfactant while an $\alpha$ =1.0 indicates the composition contains 100% silylated surfactant and no (0%) co-surfactant Mixtures of the two components are represented by the weight fraction $\alpha$, where $\alpha$ ranges as follows: $0 \leq \alpha \leq 1.0$. By example when $\alpha$ = 0.25 this indicates the surfactant mixture is composed of 25% silylated surfactant and 75% co-surfactant These blends are then diluted in water to the desired concentration for spreading evaluation.

Spreading was determined as described in Example 5, at 0.1wt% total surfactant

The silylated surfactant alone at relative concentrations (i.e. $\alpha$ = 0.75 is equivalent to 0.075% of this surfactant in water) was used as a baseline for spread performance, since the major contributor to spreading comes from the silylated surfactant The maximum spreading provided by the co-surfactant at 0.1%. ($\alpha$ = 0). Synergy is demonstrated when the blend of silylated surfactant and co-surfactant exceeds the spreading of the co-surfactant ($\alpha$ = 0) and the silylated surfactant at the relative $\alpha$ value.

Table 11 demonstrates that representative examples of the co-surfactants of the present invention provide favorable spreading results, and in some cases provide an unexpected synergistic enhancement, where the spread diameter of the mixture exceeds that of the individual components.

Table 10 - Description of Conventional Co-surfactants

| ID | Description |
|---|---|
| PAO-20 | Polyoxyethylene/polyoxypropylene copolymer (20% EO) |
| IDA-6 | Isodecyl alcohol ethoxylate (5-6 EO) |
| Oxo-TDA-5 | Oxo-tridecyl alcohol ethoxylate (5 EO) |
| APG | $C_{8-10}$ Alkylpolyglucoside |

Table 11 - Effect of Co-surfactants on Blend Spreading Properties

| | | Wt Fraction($\alpha$)Silylated Surfactant Spread diameter (mm) | | | | | |
|---|---|---|---|---|---|---|---|
| Run | Silylated Surfactan | 0 | 0.25 | 0.50 | 0.75 | 1.0 | Co-surfactant |
| 1 | 2 | 6 | 21 | 32 | 40 | 44 | PAO-20 |
| 2 | 2 | 8 | 26 | 35 | 40 | 44 | IDA-6 |
| 3 | 2 | 24 | 41 | 43 | 45 | 44 | Oxo-TDA-5 |
| 4 | 2 | 7 | 21 | 35 | 38 | 44 | APG |
| 5 | 2 | NA | 13 | 26 | 34 | 44 | None[a] |

a. Silylated Surfactant 2 alone at relative concentration (i.e.$\alpha$ = 0.25 is 0.025% product 2).

## Claims

1. An agrochemical composition comprising a silicon containing compound having the formula (X):

$$(R^1)(R^2)(R^3)S_i\ -R^4-\ Si(R^5)(R^6)(R^7) \qquad (X)$$

wherein

$R^1$, $R^2$, $R^3$, $R^5$, and $R^6$ are each independently selected from the group consisting of 1 to 6 carbon atom monovalent hydrocarbon radicals, and a hydrocarbon group of 7 to 10 carbons containing an aryl group;
$R^4$ is a hydrocarbon group of 1 to 3 carbons;
$R^7$ is an alkyleneoxide group of the general formula:

$$R^8(C_2H_4O)_d(C_3H_6O)_e(C_4H_8O)_fR^9$$

where
$R^8$ is a divalent linear or branched hydrocarbon radical having the structure:

$$-CH_2-CH(R^{10})(R^{11})_gO-$$

where

$R^{10}$ is H or methyl;
$R^{11}$ is a divalent alkyl radical of 1 to 6 carbons where the subscript g is O or 1;
$R^9$ is selected from the group consisting of H, monovalent hydrocarbon radicals of 1 to 6 carbon atoms and acetyl,
subject to the limitation that the subscripts d, e and f are 0 or positive and satisfy the relationships $2 \leq d + e + f \leq 20$ and $d \geq 2$.

2. The composition of claim 1 wherein $R^1$, $R^2$, $R^3$, $R^5$, and $R^6$ are methyl.

3. The composition of claim 2 wherein $R^{10}$ is hydrogen.

4. The composition of claim 2 wherein $R^{10}$ is methyl.

5. The composition of claim 3 or 4 wherein the subscript g is 0.

6. The composition of claim 3 or 4 wherein the subscript g is 1.

7. The composition of claim 6 wherein $R^{11}$ is $-CH_2-$.

8. The composition of claim 6 wherein $R^{11}$ is a divalent alkyl radical of 2 carbons.

9. The composition of any one of claims 1 to 8 wherein the agrochemical composition is a pesticide composition.

10. A method of formulating an agrochemical composition comprising utilizing a silicone containing compound having the formula (X):

$$(R^1)(R^2)(R^3)Si\ -R^4\ -\ Si(R^5)(R^6)(R^7) \qquad (X)$$

wherein $R^1$, $R^2$, $R^3$, $R^4$, $R^5$, $R^6$ and $R^7$ are defined as in any one of claims 1 to 8.

## Patentansprüche

1. Agrochemische Zusammensetzung, welche eine silizium haltige Verbindung mit der Formel (X):

$$(R^1)R^2)(R^3)Si\ -R^4\ -\ Si(R^5)(R^6)(R^7) \qquad (X)$$

umfasst,
wobei

$R^1$, $R^2$, $R^3$, $R^5$ und $R^6$ jeweils unabhängig aus der Gruppe bestehend aus monovalenten Kohlenwasserstoffradikalen mit 1 bis 6 Kohlenstoffatomen und einer Kohlenwasserstoffgruppe mit 7 bis 10 Kohlenstoffen, die eine Arylgruppe enthält, gewählt sind;
$R^4$ eine Kohlenwasserstoffgruppe mit 1 bis 3 Kohlenstoffen ist;
$R^7$ eine Alkylenoxidgruppe der allgemeinen Formel:

$$R^8(C_2H_4O)_d(C_3H_6O)_e(C_4H_8O)_fR^9$$

ist,
wobei $R^8$ ein bivalentes lineares oder verzweigtes Kohlenwasserstoffradikal mit der Struktur:

$$-CH_2-CH(R^{10})(R^{11})_gO-$$

ist,

wobei $R^{10}$ H oder Methyl ist;
$R^{11}$ ein bivalentes Alkylradikal mit 1 bis 6 Kohlenstoffen ist, wobei die Tieferstellung g 0 oder 1 ist;
$R^9$ gewählt ist aus der Gruppe bestehend aus H, monovalenten Kohlenwasserstoffradikalen mit 1 bis 6 Kohlenstoffatomen und Acetyl,
vorbehaltlich der Einschränkung, dass die Tieferstellungen d, e und f 0 oder positiv sind und die Beziehungen $2 \leq d + e + f \leq 20$ und $d \geq 2$ erfüllen.

**2.** Zusammensetzung nach Anspruch 1, wobei $R^1$, $R^2$, $R^3$, $R^5$ und $R^6$ Methyl sind.

**3.** Zusammensetzung nach Anspruch 2, wobei $R^{10}$ Wasserstoff ist.

**4.** Zusammensetzung nach Anspruch 2, wobei $R^{10}$ Methyl ist.

**5.** Zusammensetzung nach Anspruch 3 oder 4, wobei die Tieferstellung g 0 ist.

**6.** Zusammensetzung nach Anspruch 3 oder 4, wobei die Tieferstellung g 1 ist.

**7.** Zusammensetzung nach Anspruch 6, wobei $R^{11}$ $-CH_2-$ ist.

**8.** Zusammensetzung nach Anspruch 6, wobei $R^{11}$ ein bivalentes Alkylradikal mit 2 Kohlenstoffen ist.

**9.** Zusammensetzung nach einem der Ansprüche 1 bis 8, wobei die agrochemische Zusammensetzung eine Pestizidzusammensetzung ist.

**10.** Verfahren zum Formulieren einer agrochemischen Zusammensetzung, welche das Nutzen einer silikonhaltigen Verbindung mit der Formel (X):

$$(R^1)R^2)(R^3)Si -R^4 - Si(R^5)(R^6)(R^7) \qquad (X)$$

umfasst,
wobei $R^1$, $R^2$, $R^3$, $R^4$, $R^5$, $R^6$ und $R^7$ jeweils nach einem der Ansprüche 1 bis 8 festgelegt sind.

## Revendications

**1.** Composition agrochimique comprenant un composé contenant du silicium, de formule (X) :

$$(R^1)(R^2)\ (R^3)\ Si\text{-}R^4\text{-}Si\ (R^5)\ (R^6)\ (R^7) \qquad (X)$$

dans laquelle

chacun de $R^1$, $R^2$, $R^3$, $R^5$ et $R^6$ est indépendamment choisi dans le groupe constitué par les radicaux hydrocarbonés monovalents ayant de 1 à 6 atomes de carbone, et les groupes hydrocarbonés ayant de 7 à 10 carbones et contenant un groupe aryle ;
$R^4$ est un groupe hydrocarboné ayant de 1 à 3 carbones ;
$R^7$ est un groupe oxyalkylène de formule générale :

$$R^8(C_2H_4O)_d(C_3H_6O)_e(C_4H_8O)_fR^9$$

dans laquelle
$R^8$ est un radical hydrocarboné divalent linéaire ou ramifié de structure :

$$-CH_2-CH(R^{10})\ (R^{11})_gO-$$

dans laquelle

$R^{10}$ est H ou méthyle ;
$R^{11}$ est un radical alkyle divalent ayant de 1 à 6 carbones et l'indice g vaut 0 ou 1;
$R^9$ est choisi dans le groupe constitué par H, les radicaux hydrocarbonés monovalents ayant de 1 à 6 atomes de carbone et acétyle,
sous réserve que les indices d, e et f valent 0 ou soient positifs et satisfassent aux relations $2 \leq d + e + f \leq 20$ et $d \geq 2$.

**2.** Composition selon la revendication 1, dans laquelle $R^1$, $R^2$, $R^3$, $R^5$ et $R^6$ sont méthyle.

**3.** Composition selon la revendication 2, dans laquelle $R^{10}$ est l'hydrogène.

**4.** Composition selon la revendication 2, dans laquelle $R^{10}$ est méthyle.

**5.** Composition selon la revendication 3 ou 4, dans laquelle l'indice g vaut 0.

**6.** Composition selon la revendication 3 ou 4, dans laquelle l'indice g vaut 1.

**7.** Composition selon la revendication 6, dans laquelle $R^{11}$ est $-CH_2-$.

**8.** Composition selon la revendication 6, dans laquelle $R^{11}$ est un radical alkyle divalent ayant 2 carbones.

**9.** Composition selon l'une quelconque des revendications 1 à 8, laquelle composition agrochimique est une composition pesticide.

**10.** Procédé pour formuler une composition agrochimique, comprenant l'utilisation d'un composé contenant du silicium de formule (X) :

$$(R^1)\ (R^2)\ (R^3)Si-R^4-Si(R^5)\ (R^6)\ (R^7) \qquad (X)$$

dans laquelle $R^1$, $R^2$, $R^3$, $R^4$, $R^5$, $R^6$ et $R^7$ sont tels que définis dans l'une quelconque des revendications 1 à 8.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3159601 A
- US 3220972 A
- US 3715334 A
- US 3775452 A
- US 3814730 A
- US 5674832 A
- US 5558806 A
- US 3299112 A

### Non-patent literature cited in the description

- Homogeneous Catalysis of Hydrosilation by Transition Metals. **J.L. SPIER.** Advances in Organometallic Chemistry. Academic Press, 1979, vol. 17, 407-447